# EUROPÄISCHE PATENTSCHRIFT

(11) **EP 0 538 337 B1**
(45) Veröffentlichungstag und Bekanntmachung des Hinweises auf die Patenterteilung: **05.10.1994**
(21) Anmeldenummer: 91912838.9
(22) Anmeldetag: 10.07.1991
(51) Int. Cl.: A61K 9/06, A61K 31/27

(54) **ARZNEIMITTEL ZUR SENKUNG DES AUGENINNENDRUCKS**
DRUG FOR REDUCING INTRAOCULAR PRESSURE
MEDICAMENT POUR REDUIRE LA PRESSION INTRA-OCULAIRE

(30) Priorität: 10.07.1990 DE 4021885
(43) Veröffentlichungstag der Anmeldung: 28.04.1993
(73) Patentinhaber: Gramer, Eugen, Prof.Dr.med.Dr.jur., D-97080 Würzburg (DE)
(72) Erfinder: GRAMER, Eugen, D-8700 Würzburg (DE)
(74) Vertreter: von Füner, Alexander, Dr.
(86) Internationale Anmeldenummer: EP9101295
(87) Internationale Veröffentlichungsnummer: WO9200726

(56) Entgegenhaltungen:
- Dictionnaire Vidal, edition 52, 1976, O.V.P. (Paris, FR), see page 898 "Isopto carbachol 1.5 % and 3 %
- Unlisted Drugs, vol. 37, n 12, December 1985, (Chatham, New Jersey, US) see page 235n "Normoglaucon"

## Beschreibung

Die Erfindung bezieht sich auf ein Arzneimittel zur lokalen Anwendung am Auge zur Senkung des krankhaft erhöhten Augeninnendrucks bei Glaukom und betrifft ein Carbachol-Betablocker-Kombinationspräparat mit nur 2 x täglicher Applikation.

Das erfindungsgemäße Kombinationspräparat aus Carbachol und Betablocker kann erforderlichenfalls durch seine uneingeschränkte Kombinierbarkeit mit Dipivalyl-Epinephrin (DPE) im Rahmen einer medikamentösen Vierkomponententherapie eingesetzt werden. Ein Teil der Vierkomponententherapie kann z.B. aus der freien oder fixen Kombination eines Betablockers mit einem Miotikum, also in der Kombination z.B. von Betablocker und Pilocarpin oder Betablocker und Carbachol bestehen. Betablocker reduzieren die Kammerwassersekretion, während das Miotikum eine Verbesserung der Abflußleichtigkeit bewirkt.

Wird eine freie Carbachol-Betablocker-Kombination (Carbachol-Augentropfen 3% 4 x täglich und Betablocker 0,5% 2 x täglich) (Baseline) kombiniert mit dem Kombinationspräparat aus Dipivalyl-Epinephrin und Guanethidin (Thilodigon^{R}), so ergibt sich nach eigenen Untersuchungen ein überraschend hoher additiver drucksenkender Effekt von 4 mm Hg am 2. Tag nach dieser zusätzlichen Therapie mit dem DPE-Kombinationspräparat. Guanethidin führt zusätzlich infolge pharmakologischer Sympathektomie zur Sensibilisierung der Adrenalin-Rezeptoren und damit zur Wirkungsverstärkung des DPE.

Dieses Therapieschema, das zur maximalen Augeninnendrucksenkung bei schwer einstelllbarem Glaukom sinnvoll wäre, erfordert bisher 3 Medikamentenfläschchen, wobei Carbachol 4 x täglich, Betablocker 2 x täglich und Thilodigon^{R} 2 x täglich appliziert werden müssen. Ein solches Therapieschema ist unter Compliance-Gesichtspunkten nur begrenzt einsetzbar, da bisher ein Betablocker-Carbachol-Kombinationspräparat mit nur 2 x täglicher Applikation nicht zur Verfügung steht. Durch das erfindungsgemäße Kombinationspräparat aus Carbachol und Betablocker vereinfacht sich nun die Vierkomponententherapie auf: Carbachol-Betablocker-Kombinationspräparat (Fläschchen Nr. 1) 2 x täglich und Thilodigon^{R}-Augentropfen (Fläschchen Nr. 2) 1 x täglich oder 2 x täglich.

Zur Verfügung steht bisher ein Miotikum-Kombinationspräparat aus Pilocarpin 2% und Metipranolol 0,1% (Normoglaucon^{R}), das jedoch ebenfalls 4 x täglich appliziert werden muß. Jedoch ist ein Miotikum-Betablocker-Kombinationspräparat mit einer konstanten Drucksenkung über 12 Stunden in der Eintropfenkurve, das somit bei nur 2 x täglicher Anwendung eingesetzt werden kann und dessen Betablockerkomponente eine uneingeschränkte Kombinierbarkeit mit Dipivalyl-Epinephrin (DPE) aufweist, bisher nicht beschrieben.

Das ideale Miotikum-Betablocker-Kombinationspräparat muß somit folgende Bedingungen aufweisen:
1. eine Zweimalapplikation pro Tag,
2. eine uneingeschränkte Kombinierbarkeit mit DPE und DPE-Kombinationspräparaten und
3. eine möglichst niedrige Konzentration der Betablockerkomponente (mit 2 x täglicher Anwendung).

Der Erfindung liegt die Aufgabe zugrunde, ein entsprechendes Arzneimittel (Kombinationspräparat aus Carbachol und Betablocker) zur Verfügung zu stellen, mit dem im Vergleich zur Gabe der Einzelpräparate eine verstärkte Senkung des Augeninnendrucks erreicht werden kann, wobei die Handhabung vereinfacht ist (Applikation nur noch 2 x täglich statt bisher 4 x täglich plus 2 x täglich), die Gefahr fehlerhafter Anwendung geringer ist (nur noch 1 Fläschchen; verringerte Gesamtmenge an Wirkstoff und Konservierungsmittel bei gleicher augeninnendrucksenkender Wirkung durch geänderte Pharmakokinetik und geänderte Galenik bei fixer Kombination; Anfallsprophylaxe durch überwiegende Miotikawirkung), die Nebenwirkungen reduziert sind (verringerte Visusschwankungen, Konservierungsmittelbelastung und Gesamtmenge der Wirksubstanz) und die Kombinationsfähigkeit mit allen anderen Wirkstoffen, insbesondere mit DPE und DPE-Kombinationspräparaten, nicht eingeschränkt ist.

Im diese Vorteile aufweisenden erfindungsgemäßen Kombinationspräparat aus Carbachol und Betablocker kann grundsätzlich jeder lokal applizierbare Betablocker Anwendung finden, und zwar in Kombination mit Carbachol (Carbachol-Dosierungen 0,75%, 1,5%, 2,25% und 3%). Ein Carbachol-Betaxolol-Kombinationspräparat wird als Voraussetzung für die uneingeschränkte Kombinierbarkeit mit DPE und DPE-Kombinationspräparaten angesehen. Bei nicht selektiven Betablockern in freier Kombination mit DPE tritt ein begrenzter Wirkungsverlust bei DPE auf.

Carbachol, ein synthetisches Cholinderivat, unterscheidet sich von Acetylcholin nur dadurch, daß die Acetylgruppe durch eine Carbamyl-Gruppe ersetzt ist. Dadurch entsteht eine Doppelwirkung. Es wirkt als direktes Parasypathikomimetikum und als Cholinesterasehemmer. Diese Pharmakokinetik ist es, die diese alte Substanz als Bestandteil einer Miotikum-Betablocker-Kombination im Rahmen eines Kombinationspräparates mit erstmals nur 2 x täglicher Anwendung interessant macht und deutliche Vorteile gegenüber der Pilocarpin-Betablocker-Kombination (z.B. Normoglaucon^{R}) aufweist:
Eine Kombination des Betablockers mit Pilocarpin 2% in wässriger Lösung erfordert in Anbetracht der kürzeren Wirkungsdauer von Pilocarpin 2% ein hohes Dosierungsschema mit 4 Applikationen täglich. Bei 4 Applikationen besteht eine größere Gefahr der Allergisierung, eine größere Konservierungsmittelbelastung durch die Daueranwendung von Pilocarpin und auch eine höhere Gefahr der Ausbildung einer Subsensitivität gegenüber Pilocarpin. Durch die oben angesprochene Doppelwirkung hat Carbachol eine deutlich höhere Wirkungsdauer: So ist von Carbachol 3%-Lösung als Monosubstanz bekannt, daß die Wirkungsdauer derjenigen von Pilocarpin-Augensalbe 2% mit einer Wirkungsdauer von 12 Stunden entspricht. Im Ausmaß der Augeninnendrucksenkung entsprechen Carbachol-Augentropfen 3% der Wirkung von Pilocarpin-Augentropfen 8%. Die verlängerte Wirkungsdauer und die höhere Drucksenkung von Carbachol-Augentropfen 3% wird somit mit höherer Wahrscheinlichkeit als bei Anwendung von Pilocarpin 2% eine Reduzierung des Dosierungsschemas auf 2 x täglich erlauben. Das Therapieschema läßt sich dann besser an den spezifischen Tagesablauf des Patienten anpassen und führt so zu einer erheblichen Verbesserung der Lebensgualität für den Patienten.

Die fixe Kombination von Carbachol und Betaxolol hat auch eine längere Wirkungsdauer und eine höhere Drucksenkung zur Folge als die Kombination der Einzelsubstanzen. Der Wirkungsmechanismus des Kombinationspräparates scheint somit nicht nur die Summe der Einzelmechanismen der Monopräparate zu sein - ein überraschendes Phänomen, für das bisher noch keine gesicherte pharmakologische Klärung gefunden werden konnte. Die verlängerte drucksenkende Wirkung des Kombinationspräparates läßt sich nicht nur durch eine Reduzierung des gegenseitigen Auswascheffektes erklären, sondern läßt auch eine Änderung der Pharmakokinetik und/oder eine andere Interaktion der Medikamente im Auge vermuten. Durch die Reduzierung des Kammerwasserflusses durch die Betablockerkomponente entfaltet die Carbacholkomponente, die als direktes Parasypathikomimetikum als Cholinesterasehemmer wirkt, eine höhere Wirkungsdauer.

Das erfindungsgemäße Kombinationspräparat aus Betaxolol 0,25% Suspension (S 0,25%) oder Betaxolol 0,5% Lösung in Kombination mit Carbachol-Augentropfen 3% (bei Lösung) und einer niedrigen Konzentration bei Anwendung der Suspension, hat einen pH-Wert von 6,9 und zeigt eine gute Stabilität, ohne daß ein teures Zweikomponentenfläschen benötigt wird. Carbachol hat eine dosisabhängige Wirkung. Nur die Carbachol 3%-Lösung zeigt dabei eine signifikante Augeninnendrucksenkung über mehr als 8 Stunden. Wird Carbachol als Kombinationspräparat mit Betaxolol als Suspension gegeben, so kann bei gleicher Wirkung die Konzentration reduziert werden. Für die Betablockerkomponente in Suspension ist gesichert, daß dieselbe augeninnendrucksenkende Wirkung mit nahezu halbierter Konzentration, also mit S 0,28%-igem Betaxolol (bei Anwendung der Suspensionstechnik) erreicht wird, wie mit 0,5 % der Betaxolol-Lösung. Bei Einmalapplikation von 1,5%-iger Carbachol-Lösung läßt sich eine konstante Miosis über fast 24 Stunden erreichen. Visusschwankungen sind daher bei der Carbachol-Kombination im Gegensatz zur Pilocarpin-Kombination weniger ausgeprägt zu erwarten. Durch Anwendung der Suspensionstechnologie, die sowohl dem Betablocker, als auch dem Carbachol den Bolus-Charakter durch die verzögerte Abgabe der Wirksubstanz nimmt, lassen sich lokale und systemische Nebenwirkungen reduzieren. Durch die Reduzierung der Wirstoffgesamtmenge bei Anwendung der Suspensionstechnologie (nahezu Halbierung der Konzentration beim Betablocker und Reduzierung der Konzentration beim Carbachol-Präparat) und der zusätzlichen Reduzierung der Tropfenhäufigkeit auf 2 x täglich läßt sich mit dem Kombinationspräparat ein höheres Sicherheitsprofil hinsichtlich systemischer Nebenwirkungen erreichen.

Um wieviel die Konzentration des Carbachols bei Anwendung der Suspensionstechnologie gesenkt werden kann bei gleicher Augeninnendrucksenkung wie Carbachol 3%-Lösung, wird derzeit u.a. tierexperimentell an Cynomoglus-Affen geprüft. Die Wahl des Betablockers Betaxolol hat, neben den geringeren pulmonalen Nebenwirkungen dieses Betablockers im Vergleich zu anderen, auch eine bessere Kombinierbarkeit des neuen Carbachol-Betaxolol-Kombinationspräparats mit anderen Wirkstoffgruppen zur Folge: Die Abflußverbesserung des Adrenalins wird durch unspezifische Betablocker zwar nicht aufgehoben, kann jedoch reduziert sein, wobei Betaxolol, ein Beta-1-spezifischer Blocker, tierexperimentell an Primaten diese Wirkungsreduzierung des DPE bei freier Kombination nicht zeigte. Daraus ergibt sich eine bessere Kombinierbarkeit des Betaxolol-Carbachol-Kombinationspräparats mit DPE und DPE-Kombinationspräparaten, wenn es in einer Mehrkomponententherapie bei sehr hohen Augeninnendruckswerten eingesetzt werden muß.

Der entscheidende Vorteil des vorgeschlagenen Carbachol-Betaxolol-Präparats ist für die wässerige Lösung wie auch für die Suspension die nur 2 x tägliche Anwendung. Die Anwendung einer Suspensionstechnologie bestehend aus Poly- (Styrol-Divinylbenzol) Sulfonsäure (Amberlite IRP-69) 2,5 mg als Wirkstoffträger und Carbomer 934 P 2 mg zur Erhöhung der Depotfunktion (ALCON-Patent) führt dazu, daß sich die Wirkstoffmenge unter Beibehaltung der 2 x täglichen Applikation bei gleicher Augeninnendrucksenkung reduzieren läßt. Neu in diesem Kombinationspräparat ist, daß die Suspensionstechnologie erstmals auch für Carbachol angewandt wird zur Penetrationsverbesserung auch beim Carbachol.

Aufgrund der bisherigen Erkenntnisse erscheint es daher zweckmäßig, im Kombinationspräparat als Lösung sowohl Carbachol wie den Betablocker in seiner national (oder regional) zugelassenen höchsten Konzentration vorzusehen, während bei Anwendung der Suspensionstechnologie Betaxolol S 0,28%-ig und Carbachol 3% bzw. Carbachol S 1,5% vogesehen werden sollten.

Die Doppelwirkung des Carbachols als direktes Parasppathikomimetikum und als Cholinesterasehemmer ist vorteilhaft in fixer Kombination mit Betablockern, z.B. mit Betaxolol. Die hydrophilen Eigenschaften des Carbachol, die durch das Konservierungsmittel Benzalkoniumchlorid wie auch durch die Suspensionstechnologie verbessert werden, sind vermutlich ein Grund dafür, daß bisher die überraschend guten Eigenschaften des Carbachols für eine fixe Kombination nicht beachtet wurden.

## Patentansprüche

1. Arzneimittel zur Senkung des Augeninnendrucks, dadurch **gekennzeichnet,** daß es sowohl Carbachol als auch einen lokal applizierbaren Betablocker enthält.

2. Arzneimittel nach Anspruch 1, dadurch **gekennzeichnet,** daß das Carbachol in der therapeutischen Konzentration von 0,5 bis 4 %, insbesondere 3 %, und der Betablocker in der therapeutischen Konzentration von 0,1 bis 2 % enthalten sind, insbesondere als Betaxolol S 0,28 % oder Betaxolol-Lösung 0,5 %.

3. Arzneimittel nach Anspruch 2, dadurch **gekennzeichnet,** daß das Carbachol in einer Konzentration von 3 % enthalten ist und als Betablocker Betaxolol mit einer Konzentration von 0,5 % (wässerige Lösung) enthalten ist, wobei bei Anwendung der Suspensionstechnologie Carbachol S in 1,5 bis 3 % und Betaxolol S in 0,28%iger Konzentration enthalten ist.

4. Arzneimittel nach einem der Ansprüche 1 bis 3, dadurch **gekennzeichnet,** daß es in Form von Augentropfen, Augensalbe, Augengel, Augeninsert oder auf einem sonstigen Medikamententräger in Suspensionstechnologie zur lokalen Applikation am Auge zubereitet ist.

## Claims

1. A medicament for reducing pressure inside the eye, characterised in that it contains a betablocker which can be applied locally and carbachol.

2. A medicament according to claim 1, characterised in that the carbachol is contained in the therapeutic concentration of 0.5 to 4 %, in particular 3 %, and the betablocker is contained in the therapeutic concentration of 0.1 to 2 %, in particular as betaxolol S 0.28 % or betaxolol solution 0.5 %.

3. A medicament according to claim 2, characterised in that the carbachol is contained in a concentration of 3 %, and as a betablocker betaxolol is contained having a concentration of 0.5 % (aqueous solution), wherein when using suspension technology carbachol S is contained in 1.5 to 3 % and betaxolol S in 0.28 % concentration.

4. A medicament according to one of claims 1 to 3, characterised in that is it prepared in the form of eye drops, eye ointment, eye gel, eye insert or another type of medicament carrying medium in suspension technology for local application to the eye.

## Revendications

1. Médicament destiné à abaisser la pression intra-oculaire, ***caractérisé en ce qu*****'**il contient à la fois du carbachol et un béta-bloquant à application locale.

2. Médicament selon la Revendication 1, ***caractérisé en ce qu***'il contient le carbachol à la concentration thérapeutique de 0,5 à 4 %, notamment de 3 % et le béta-bloquant à la concentration thérapeutique de 0,1 à 2 %, en particulier sous la forme de bétaxolol S à 0,28 % ou d'une solution de bétaxolol à 0,5 %.

3. Médicament selon la Revendication 2, ***caractérisé en ce qu***'il contient le carbachol à une concentration de 3 % et le bétaxolol, utilisé comme béta-bloquant, à une concentration de 0,5 % (solution aqueuse), cependant que, dans le cas de la technique en suspension, il contient du carbachol S à une concentration de 1,5 à 3 % et du bétaxolol S à une concentration de 0,28 %.

4. Médicament selon l'une des Revendications 1 à 3, ***caractérisé en ce qu***'il est préparé pour l'application locale sur l'oeil sous la forme de gouttes oculaires, de pâte oculaire, de gel oculaire, d'insert oculaire ou sous la forme portée par un autre support de médicament dans la technique en suspension.
